# FASCICULE DE BREVET EUROPEEN

(11) **EP 2 989 455 B1**
(45) Date de publication et mention de la délivrance du brevet: **04.01.2017**
(21) Numéro de dépôt: 14726677.9
(22) Date de dépôt: 11.04.2014
(51) Int. Cl.: G01N 33/20, G01N 3/06

(54) **PROCEDE D'ANALYSE D'UN FACIES DE RUPTURE D'UNE PIECE DE TURBOMACHINE**
VERFAHREN ZUR ANALYSE EINER FRAKTUROBERFLÄCHE EINES STRÖMUNGSMASCHINENBAUTEILS
PROCESS FOR ANALYSING A FRACTURE SURFACE OF A TURBOMACHINE PART

(30) Priorité: 22.04.2013 FR 1353660
(43) Date de publication de la demande: 02.03.2016
(73) Titulaire: Safran Aircraft Engines, 75015 Paris (FR)
(72) Inventeur: COLLADON, Fabrice, F-77550 Moissy-Cramayel cedex (FR)
(74) Mandataire: Gevers & Orès
(86) Numéro de dépôt international: PCT/FR2014/050880
(87) Numéro de publication internationale: WO 2014/174179

(56) Documents cités:
- WO-A1-2007/048934
- FR-A1- 2 968 759
- POURSAEIDI ET AL: "Failure analysis of generator rotor fan blades", ENGINEERING FAILURE ANALYSIS, PERGAMON, GB, vol. 14, no. 5, 9 février 2007 (2007-02-09), pages 851-860, XP005881368, ISSN: 1350-6307, DOI: 10.1016/J.ENGFAILANAL.2006.11.042
- WITEK ET AL: "Failure analysis of turbine disc of an aero engine", ENGINEERING FAILURE ANALYSIS, PERGAMON, GB, vol. 13, no. 1, 1 janvier 2006 (2006-01-01), pages 9-17, XP028074658, ISSN: 1350-6307, DOI: 10.1016/J.ENGFAILANAL.2004.12.028 [extrait le 2006-01-01]
- MAZUR ET AL: "Steam turbine blade failure analysis", ENGINEERING FAILURE ANALYSIS, PERGAMON, GB, vol. 15, no. 1-2, 6 septembre 2007 (2007-09-06), pages 129-141, XP022233939, ISSN: 1350-6307, DOI: 10.1016/J.ENGFAILANAL.2006.11.018

## Description

### DOMAINE TECHNIQUE

La présente invention concerne un procédé d'analyse d'un faciès de rupture ou de fissure d'une pièce métallique de turbomachine, et en particulier en alliage métallique à base de TiAl.

### ETAT DE L'ART

Des pièces de turbomachine peuvent se fissurer ou se rompre au cours de leur développement ou en service. Une analyse du plan de rupture ou de fissure (fractographie) appelé « faciès » est alors réalisée en laboratoire. Ce faciès correspond au plan de rupture d'une pièce ou au plan de fissuration avant ouverture en laboratoire pour une pièce fissurée non rompue. L'analyse du faciès doit permettre d'expliquer comment et pourquoi les pièces se sont rompues ou fissurées. Les objectifs principaux de cette analyse sont notamment de localiser la zone d'amorçage de la fissure ou rupture, d'identifier d'éventuelles particularités métallurgiques ou géométriques de la pièce, de déterminer le type de fissure ou rupture (brutale, vibratoire ou cyclique), le mode de sollicitation (flexion, torsion ou traction) de la pièce qui a provoqué la rupture ou fissure, etc.

Les faciès de rupture ou de fissure de pièces de turbomachine en alliage métallique à base de nickel ou de cobalt sont lisibles et l'analyse précitée est réalisée sans trop de difficultés car ces faciès sont relativement lisses et les zones d'amorçage de rupture ou de fissure peuvent être déduites de la forme et de l'orientation de macrostries et microstries de fatigue visibles sur le faciès.

De nouveaux alliages métalliques, notamment ceux à base de TiAl, sont utilisés pour la fabrication de pièces d'une turbomachine, ces nouveaux alliages étant aussi résistants que ceux à base nickel et ayant l'avantage d'être plus léger. Il est nécessaire d'étudier le comportement de ces nouveaux alliages à la rupture ou fissure.

Les études bibliographiques ainsi que les essais en laboratoire (traction, fatigue HCF, fatigue LCF, fluage, etc.) indiquent que ces nouveaux alliages ne réagissent pas de manière conventionnelle à une fissuration et une rupture, ce qui rend très difficile la localisation d'un site d'amorçage d'une rupture ou fissure, le sens de propagation de la rupture ou fissure ainsi que la différentiation entre une fissuration progressive en fatigue et une rupture brutale.

Dans le cas particulier de l'observation du faciès de rupture d'une aube de turbine en alliage à base de TiAl (tel que du Ti48-2-2), on constate que ce faciès est homogène et qu'il est très difficile de localiser la zone d'amorçage de la rupture, le front de propagation de cette rupture et le type de rupture.

Poursaeidi et al. "Failure analysis of generator rotor fan blades" en Engineering Failure Analysis 14(5):851-860, 2007, décrit une analyse conventionnelle d'un faciès de rupture.

Il existe donc un réel besoin d'une méthodologie d'analyse du faciès de pièces réalisées dans ces nouveaux alliages métalliques, qui soit simple, efficace et économique, et applicable dans le domaine de l'expertise d'analyses d'avarie.

### EXPOSE DE L'INVENTION

L'invention propose un procédé d'analyse d'un faciès de rupture ou de fissure d'une pièce métallique de turbomachine, en particulier en matériau à rupture totalement fragile, tel un alliage à base de TiAl, ledit faciès correspondant au plan de rupture ou au plan de fissuration avant ouverture en laboratoire pour une pièce fissurée non rompue , caractérisé en ce qu'il comprend au moins l'une des étapes consistant à :
a) repérer sur le faciès la position et l'orientation de facettes de clivage, de façon à identifier une zone d'amorçage de la rupture ou fissure et à déterminer le sens de propagation de cette rupture ou fissure,
b) examiner le faciès et détecter les zones de présence de grains équiaxes et/ou de grains lamellaires, de façon à évaluer la température à laquelle la rupture ou fissure est intervenue, et
c) comparer la ou les colorations thermiques du faciès avec celles d'échantillons d'un nuancier de coloration thermique, ces échantillons étant réalisés dans le même matériau que la pièce et étant soumis à des traitements thermiques oxydants à des températures et pendant des durées prédéterminées, de façon à évaluer la vitesse de propagation de la rupture ou fissure.

Plus le procédé selon l'invention comprend d'étapes, plus l'analyse du faciès de la pièce est complète. Le procédé peut comprendre l'étape a) seule, l'étape b) seule, l'étape c) seule, les étapes a) et b), les étapes a) et c), les étapes b) et c), ou les étapes a), b) et c).

Les étapes a), b) et/ou c) sont effectuées dans un ordre quelconque mais sont de préférence effectuées dans l'ordre présenté ci-dessus.

Le procédé selon l'invention permet de localiser précisément la zone d'amorçage de la rupture ou fissure (étape a)), de connaître la température à laquelle a lieu la rupture ou se forme la fissure (étape b)), et/ou de déterminer s'il s'agit d'une rupture brutale ou d'une fissuration progressive en fatigue (étape c)).

Dans la présente demande, on entend par matériau à rupture totalement fragile, un matériau dont une rupture ne laisse aucune marque conventionnelle analysable pour examiner l'origine de cette rupture.

L'étape a) est basée sur l'analyse de la position et de l'orientation des facettes de clivage du faciès. Les cristaux fragiles du matériau de la pièce se brisent le long de certains plans formant ces facettes. Ces facettes sont en général toutes orientées sensiblement radialement vers l'extérieur par rapport à un point central situé au niveau du front de propagation de la rupture ou fissure. Elles sont réparties sur sensiblement toutes la surface du faciès ayant subie la rupture ou fissure. La zone de rupture finale est en général située à l'opposé de la zone d'amorçage. Les facettes permettent donc de renseigner sur le sens de propagation de la rupture ou fissure.

L'étape a) est par exemple réalisée à la loupe binoculaire et/ou au moyen d'un système d'imagerie par microscopie électronique à balayage (MEB). L'étape a) peut consister à repérer et à tracer directement sur une image vidéo du faciès la position et l'orientation des facettes de clivage.

L'étape a) peut en outre consister à déterminer au moins un défaut géométrique (rayure, choc, etc.) ou métallurgique (porosité, inclusion, etc.) pouvant expliquer l'apparition de la fissure ou rupture.

Le faciès de fissure ou rupture est modifié en fonction de la température à laquelle la rupture ou fissure est intervenue. Le matériau de la pièce peut comprendre des grains équiaxes et/ou des grains lamellaires visibles sur le faciès. Les grains équiaxes n'ont pas d'orientation privilégiée (cristaux symétriques et isotropes) et apparaissent en général lorsque le matériau est soumis à une température supérieure ou égale à 500°C. La présence ou l'absence de ces grains sur un faciès de rupture permet donc d'évaluer la température à laquelle la rupture ou fissuration est intervenue. Le procédé peut ainsi consister, à l'étape b), à déterminer si la rupture ou la fissuration est intervenue à plus ou moins que 500°C. L'étape b) peut en outre consister à déterminer si la rupture ou fissuration a eu lieu à chaud en service, ou à froid en fabrication.

L'étape b) est par exemple réalisée à la loupe binoculaire et/ou au moyen d'un système d'imagerie par microscopie électronique à balayage (MEB).

L'étape b) du procédé peut consister à évaluer la température à laquelle la rupture ou fissure est intervenue par une estimation de la densité des grains équiaxes sur le faciès. En effet, l'inventeur a constaté que la densité de ces grains augmente avec la température.

Le procédé peut en outre consister, à l'étape b), à examiner le faciès et détecter les zones de présence de cupules ductiles. L'alliage TiAl est relativement fragile jusqu'à 800°C car il a un allongement à la rupture de 1 à 3%. Cet allongement augmente de manière significative à partir de 800°C et est de 20% environ à 900°C. A partir de 800°C, des cupules ductiles apparaissent sur le faciès et peuvent être facilement identifiées par observation d'une image MEB du faciès par exemple. Dans le cas ou de telles cupules sont présentent sur un faciès, cela signifie donc que la pièce a été soumise à une surchauffe anormale car cet alliage n'est en général pas utilisée à plus de 800°C dans une turbomachine car il perd des propriétés mécaniques. La détection de cupules ductiles sur un faciès permet donc d'évaluer la température à laquelle la pièce a été soumise.

L'étape c) permet de déterminer le type de rupture de la pièce, par comparaison de la ou des colorations thermiques du faciès avec celles d'échantillon d'un nuancier ou abaque préalablement préparé, comme cela est décrit dans la demande antérieure FR-A1-2 968 759 de la demanderesse. Dans le cas où le plan de rupture du faciès a une coloration uniforme, la rupture a été brutale. Au contraire, dans le cas où le plan de rupture présente un dégradé de coloration, la rupture a été évolutive et est en général due à une fatigue vibratoire ou oligocyclique. Ce type de fatigue peut être déterminé par une datation des phases de propagation de la rupture au travers des gradients de coloration thermique du faciès.

A l'étape c), avant la soumission des échantillons aux traitements thermiques, une entaille peut être réalisée dans chaque échantillon qui est ensuite soumis à des contraintes pour générer une fissure ou rupture au niveau de cette entaille.

L'étape c) peut en outre consister à comparer les colorations thermiques d'une surface de la pièce avec celles des échantillons précités, de façon à évaluer le niveau de température atteint par la pièce.

### DESCRIPTION DES FIGURES

L'invention sera mieux comprise et d'autres détails, caractéristiques et avantages de l'invention apparaîtront à la lecture de la description suivante faite à titre d'exemple non limitatif en référence aux dessins annexés dans lesquels :
- la figure 1 est une image à la loupe binoculaire d'un faciès de rupture d'une aube en TiAl de turbomachine,
- les figures 2 et 3 sont des images MEB du faciès de rupture de l'aube de la figure 1,
- la figure 4 est une image à la loupe binoculaire d'un autre faciès de rupture d'une aube en TiAl,
- la figure 5 est une image MEB d'une partie du faciès de la figure 4,
- la figure 6 est une image MEB partielle d'un autre faciès de rupture d'une aube en TiAl,
- la figure 7 est une vue très schématique en coupe d'une pièce en TiAl, et représente le cheminement d'une fissure au travers des zones granulaires de la pièce,
- la figure 8 est une image MEB partielle d'un autre faciès de rupture d'une aube en TiAl, et montre des grains équiaxes et des grains lamellaires,
- la figure 9 est une image vidéo du faciès d'une aube en TiAl ayant subi un traitement thermique d'oxydation,
- la figure 10 est une image vidéo d'un nuancier de coloration thermique,
- la figure 11 est un organigramme des différentes étapes d'un mode de réalisation du procédé selon l'invention, et
- les figures 12 à 14 sont des images MEB de faciès de rupture montrant respectivement des facettes de clivage, des grains équiaxes et des cupules ductiles.

### DESCRIPTION DETAILLEE

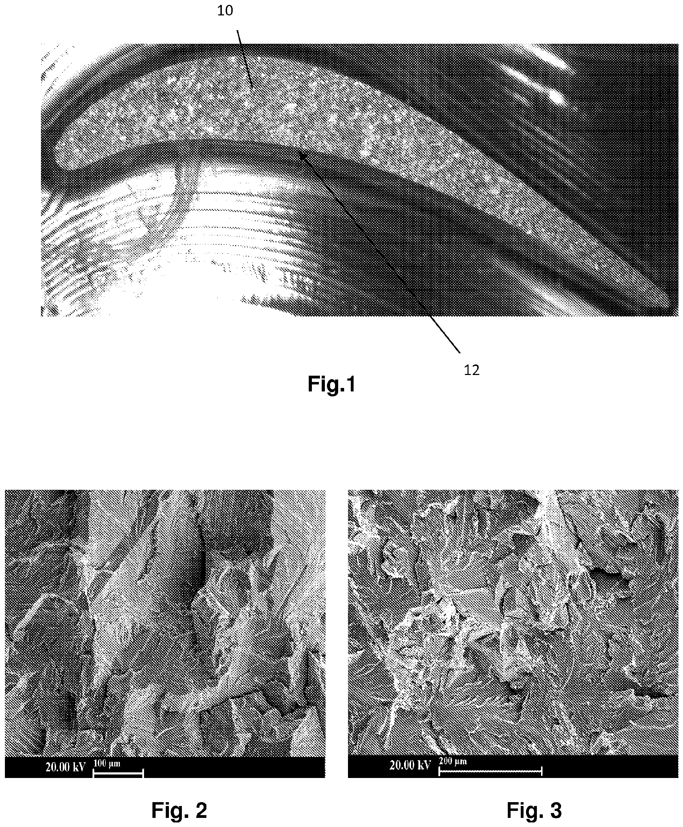

On se réfère d'abord à la figure 1 qui est une image à la loupe binoculaire d'un faciès de rupture 10 d'une aube 12 en TiAl d'une turbine de turbomachine. Le faciès de rupture 10 s'étend dans un plan qui est ici un plan transversal à la pale de l'aube.

Comme expliqué dans ce qui précède, on constate que la morphologie de rupture de ce faciès est relativement homogène et que les techniques d'analyse de faciès de rupture de pièces de turbomachine, utilisées dans la technique actuelle, ne peuvent pas être utilisées pour analyser ce faciès.

Les figures 2 et 3 sont des images MEB de faciès de rupture d'aubes de turbine en TiAl, les ruptures ayant été volontairement provoquées par des sollicitations en fatigue. Le faciès de la figure 2 est un faciès de fissure en fatigue vibratoire ou polycyclique (HCF) et celui de la figure 3 est un faciès de rupture statique. On constate une absence de différence de morphologie fractographique entre ces faciès, un constat similaire ayant été fait en fatigue oligocyclique (LCF) ou fluage.

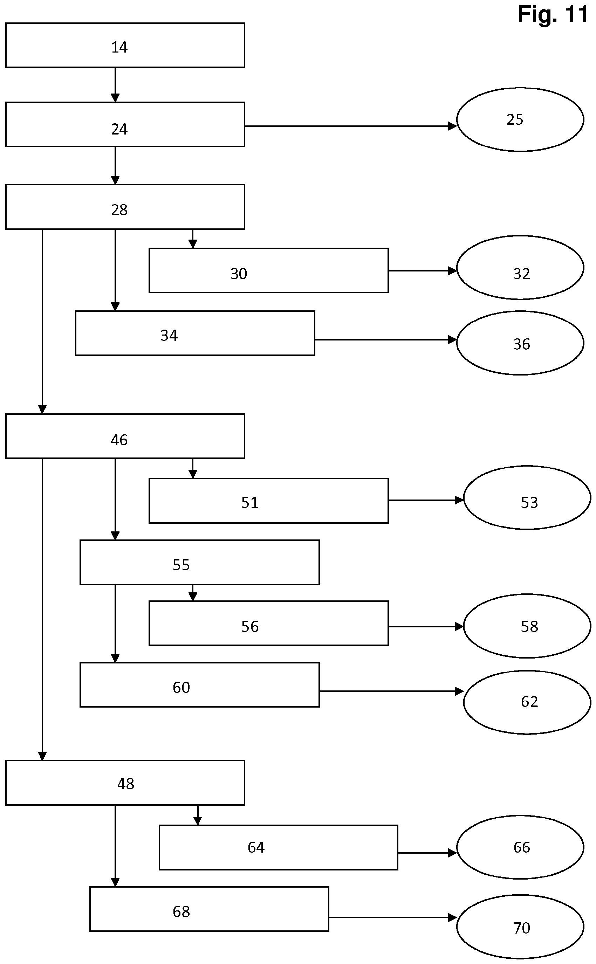

La présente invention propose un procédé d'analyse d'un faciès de rupture ou de fissure d'une pièce métallique de turbomachine, en particulier en TiAl, un mode de réalisation de ce procédé étant schématiquement représenté par l'organigramme de la figure 11.

Une première étape 14 du procédé de la figure 11 consiste à cartographier sur un faciès de rupture 10 les facettes de clivage à la loupe binoculaire, c'est-à-dire à repérer sur ce faciès la position et l'orientation des facettes de clivage. Ces facettes de clivage représentent des surfaces de rupture de grains fragiles du matériau.

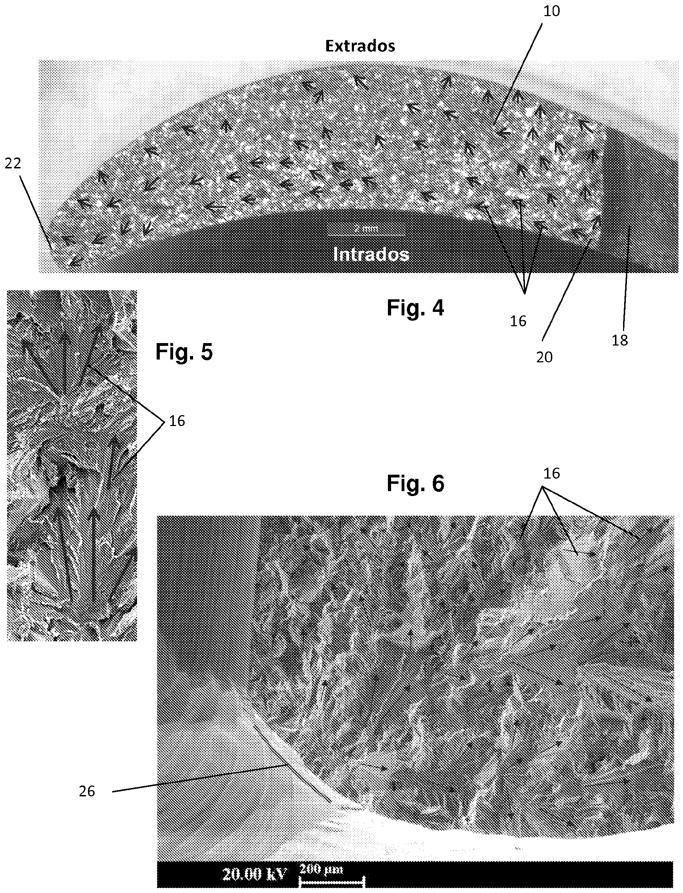

La figure 4 est une image obtenue à la loupe binoculaire et traitée informatiquement pour y apposer des flèches qui représentent la position et l'orientation des facettes de clivage 16. La figure 5 est une image MEB à plus grande échelle d'une partie de la figure 4. La rupture de l'aube de la figure 4 a été obtenue en réalisant une entaille 18 dans le bord de fuite de la pale puis en soumettant l'aube à un essai de rupture brutale provoquée en laboratoire suivant un mode de flexion, de l'intrados vers l'extrados.

L'orientation des facettes de clivage 16 est cohérente avec le sens de rupture de la pale en flexion. La seconde étape 24 du procédé de la figure 11 consiste à identifier la zone d'amorçage et à déterminer le sens de rupture (la zone de rupture finale étant située à l'opposée de la zone d'amorçage). Cette étape peut également permettre de déterminer le défaut géométrique (rayure ou choc - référence 25 en figure 11) ou métallurgique (porosité ou inclusion) qui est à l'origine de la rupture.

Dans l'exemple représenté en figure 4, les facettes de clivage 16 sont sensiblement dirigées depuis le fond de l'entaille 18 (et plus exactement au droit de l'angle vif 20 en fond d'entaille 18) jusqu'au bord d'attaque 22 de la pale. Ceci permet de localiser l'amorçage de la rupture au niveau de cet angle vif 20, et d'en conclure également que cet angle vif constitue un facteur géométrique d'apparition de la rupture (du fait de la concentration de contraintes au niveau de cet angle vif).

La figure 6 est une image MEB d'un autre faciès de rupture d'une aube en TiAl, la rupture ayant été provoquée par un essai en fatigue HCF à température ambiante. La position et l'orientation des facettes de clivage 16 permettent de localiser la zone d'amorçage 26 au niveau du bord d'attaque de l'aube.

Le TiAl est un alliage dont l'utilisation est particulièrement intéressante pour une plage de température de 600 à 800°C. Pour les turbines de turbomachine, il est utilisé dans les étages basse pression à des températures moyennes de 750°C. Le procédé selon l'invention utilise les propriétés d'évolution cristallographique du matériau en fonction de la température à laquelle il est soumis.

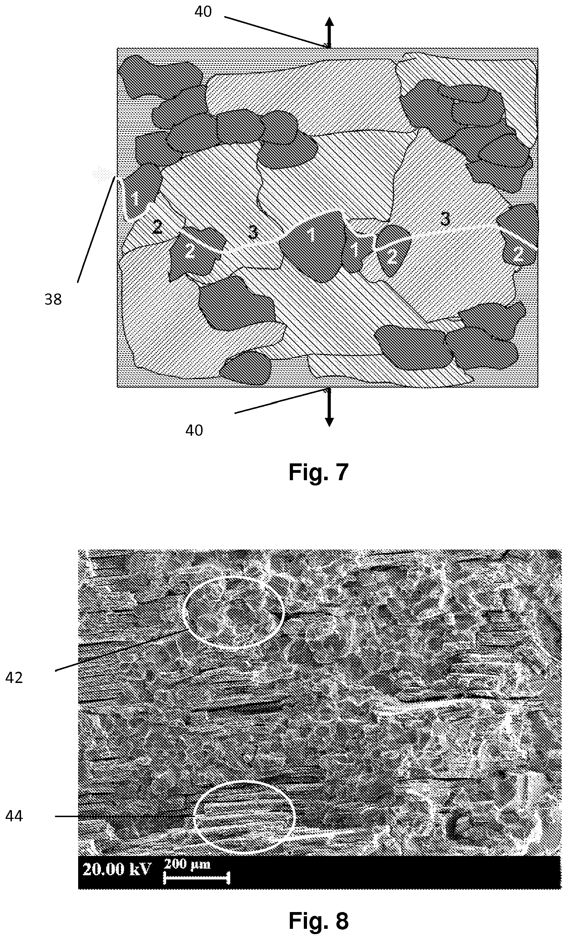

La figure 7 est une vue très schématique en coupe d'une pièce en TiAl dans lequel s'est propagée une fissure dont le cheminement est indiqué par la référence 38. Cette fissure a été générée par un essai en traction (flèches 40) dans le plan de coupe du dessin. Dans ce dessin schématique, chaque zone délimitée par un trait continu définit une zone dans laquelle est présente un type de grains particuliers. Les zones en gris comprennent des grains équiaxes γ et les zones hachurées comprennent des grains lamellaires γ+α2. La pièce présente donc une microstructure duplex. De plus, le chiffre « 1 » indique que la fissure fait le tour de grains lamellaires, ce qui se traduit par la présence de motifs intergranulaires en surface du faciès de fissure, le chiffre « 2 » indique que la fissure traverse des grains équiaxes ou lamellaires ce qui se traduit par la présence de motifs de clivage en surface du faciès (la fissure se propageant en parallèle des lamelles dans le cas des grains lamellaires), et le chiffre « 3 » indique que la fissure traverse des grains lamellaires dans une direction non parallèle aux lamelles. La propagation de la fissure à travers des grains équiaxes ou lamellaires (références 2 et 3) fait apparaître des facettes de clivage sur le faciès de rupture. Les mêmes résultats sont obtenus lorsque la pièce est soumise à un essai en fatigue vibratoire à fréquence élevée (HCF) ou en fatigue oligocyclique à faible fréquence(LCF). Des résultats différents sont toutefois apparus en fonction de la température de traitement thermique de la pièce. Les grains équiaxes sont absents sur les plans de rupture lorsque la pièce n'est pas exposée thermiquement. Ces grains commencent à apparaître à partir de 500°C et leur densité augmente à mesure que la température d'exposition augmente. Une pièce soumise à un traitement thermique de 700°C et rompue à cette température comprend deux à trois fois plus de grains équiaxes qu'une pièce soumise rompue soumise à un traitement thermique de 500°C. L'analyse de la densité de grains équiaxes sur un faciès de rupture ou fissure permet donc également d'évaluer la température vue par la pièce.

Une troisième étape 28 du procédé de la figure 11 consiste à examiner une image MEB du faciès en début, au milieu et en fin de rupture. Dans le cas où cet examen révèle la présence de motifs intergranulaires (grains équiaxes - référence 30 en figure 11) sur le faciès, cela signifie que la fissure ou rupture a eu lieu à chaud (c'est-à-dire en service - référence 32 en figure 11) car ces motifs n'apparaissent que lorsque le matériau est soumis à une température supérieure à 500°C. Dans le cas où l'examen révèle l'absence de tels motifs (référence 34 en figure 11), cela signifie que la fissure ou rupture a eu lieu à froid (référence 36 en figure 11), c'est-à-dire lors de la fabrication de la pièce.

Du fait de l'apparition des grains équiaxes à partir de 500°C, la présence ou l'absence de ces grains sur un faciès de rupture ou de fissure permet de déterminer le niveau de température à laquelle s'effectue la fissuration ou rupture. On peut donc déterminer à quel stade sont apparues des fissures, soit en fabrication (dans le cas par exemple d'un usinage de la pièce à température ambiante - il n'y a alors pas de grains équiaxes sur le plan de rupture mais seulement des grains clivés), soit en fonctionnement dans la turbine (à chaud - la différentiation des différents grains étant alors relativement aisée).

Les dernières étapes 46, 48 du procédé de la figure 11 permettent d'une part de déterminer le niveau de température atteint par la pièce et d'autre part d'évaluer la vitesse de propagation de la rupture ou fissure.

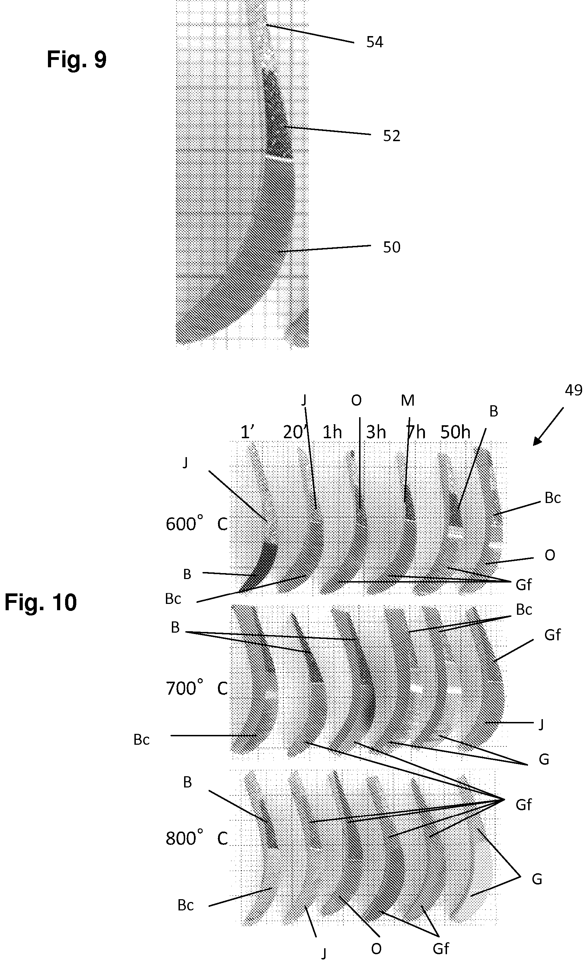

La figure 9 montre le faciès de fissure d'une aube qui a subi un traitement thermique d'oxydation. Une entaille 50 a été réalisée dans la pale de l'aube qui a ensuite été fissurée par un essai en fatigue. La zone 52 du faciès représente la partie fissurée et la zone 54 représente une partie découpée après traitement thermique d'oxydation pour observer le faciès. Seule la partie entaillée 50 et la zone fissurée sont colorées car seules ces parties étaient exposées à l'oxydation. Cette oxydation a entraîné la formation d'une couche d'oxyde dans les zones 50, 52, qui se traduit par une coloration thermique superficielle de ces zones, cette coloration étant fonction de la température et de la durée du traitement thermique.

Un nuancier de coloration thermique du matériau de l'aube est préparé en classant dans ce nuancier des échantillons ayant subi des traitements thermiques, en fonction de leur température et de leur durée de traitement.

Pour cela, plusieurs échantillons réalisés dans un matériau identique à celui de l'aube sont préparés (entaillés et fissurés) et subissent un traitement thermique, comme expliqué dans ce qui précède en référence à la figure 9. Dans l'exemple de la figure 10, trois séries d'échantillons sont préparées, chaque série comportant six échantillons soit un total de dix-huit échantillons.

Ces échantillons sont ensuite soumis à des traitements thermiques oxydants différents, le traitement thermique de chaque échantillon étant différent des traitements thermiques des autres échantillons par sa température et/ou sa durée de traitement. Les traitements thermiques peuvent être réalisés au moyen d'un four dans lequel sont introduits les échantillons qui sont chacun équipés d'un thermocouple relié à des moyens appropriés pour mesurer leur température de traitement. Le four est alimenté en air ambiant. Les trois séries d'échantillons sont soumises à des températures de traitement de 600, 700 et 800°C, respectivement, et les échantillons de chaque série sont soumis à des durées de traitement de 1 min, 20min, 1h, 3h, 7h et 50h, respectivement.
Des colorations thermiques superficielles apparaissent sur au moins une partie des échantillons. Ces colorations sont plus ou moins prononcées et peuvent être plutôt jaune (J), ocre (0), marron (M), bleue (B), bleue clair (Bc), gris (G) ou gris foncé (Gf). Ces colorations dépendent notamment de la nature et de l'épaisseur de la couche d'oxyde qui se forme sur les échantillons lors du traitement thermique.

La figure 10 représente un nuancier 49 comportant les dix-huit échantillons précités, c'est-à-dire les trois séries de six échantillons. Les échantillons sont fixés sur un support pour former un tableau dans lequel chaque ligne correspond à une température de traitement et chaque colonne correspond à une durée de traitement. Les températures de traitement sont classées de haut en bas par ordre croissant et les durées de traitement sont classées de gauche à droite par ordre croissant.

Les colorations thermiques superficielles des échantillons ont été schématiquement représentées par les lettres J, O, M, B, Bc, G et Gf qui désignent respectivement des nuances de jaune, ocre, marron, bleu, bleu clair, gris ou gris foncé. Lorsque deux échantillons présentent des colorations similaires, les niveaux de matité ou de brillance de ces échantillons peuvent être différents et donc permettre de distinguer les échantillons les uns des autres. Par ailleurs, des écaillages des surfaces des échantillons peuvent apparaître. Le nuancier révèle clairement que l'évolution de la coloration thermique superficielle d'une pièce est fonction à la fois du niveau de température atteint et du temps de maintien à cette température. Il est préférable de comparer les colorations thermiques d'une pièce, directement avec les échantillons d'un nuancier et non pas avec des clichés photographiques de ce nuancier car les couleurs des échantillons peuvent être différentes sur ces clichés.

Ce nuancier permet d'une part, à partir de la coloration thermique superficielle d'une aube, de déterminer le niveau de température atteint par l'aube par comparaison de cette coloration avec celles des échantillons du nuancier (étape 46).

Dans un premier temps (étape 51), la coloration thermique de l'intrados ou de l'extrados de la pale de l'aube peut être comparée à celles des échantillons du nuancier, de façon à évaluer le niveau de température 53 atteint par l'aube.

Ensuite (étape 55), la coloration thermique du faciès de rupture peut être comparée à celles des échantillons du nuancier. Dans le cas où cette coloration est uniforme 56, on peut en conclure que la rupture a été brutale 58. Dans le cas contraire où le faciès comprend un dégradé de coloration 60, on peut en conclure que la rupture a été évolutive 62 et est due à des sollicitations en fatigue.

Le nuancier permet d'autre part d'évaluer la vitesse de propagation de la fissure ou rupture au travers des gradients d'oxydation de coloration thermique du faciès (étape 48). En effet, dans le cas précité où le faciès comprend un dégradé ou gradient de coloration, la comparaison de ces colorations avec celles des échantillons du nuancier permet de déterminer si l'évolution de la propagation a été rapide 64 ou lente 68. Une vitesse de propagation rapide 64 signifie que la rupture est due à une fatigue vibratoire (HCF - 66) et une vitesse de propagation lente 68 signifie qu'elle est due à une fatigue oligocyclique (LCF - 70).

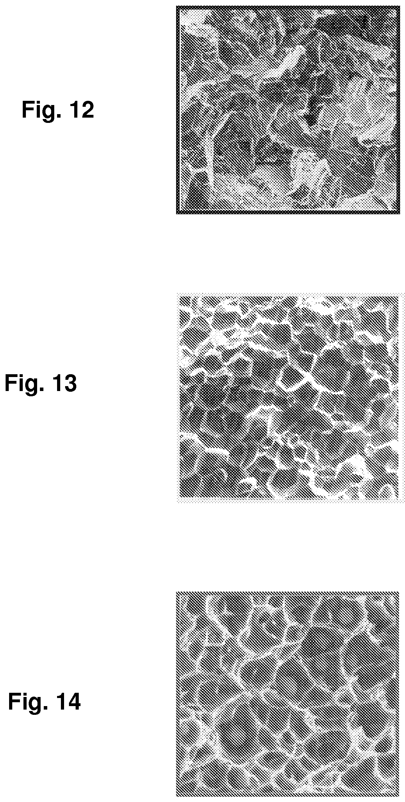

Les figures 12 à 14 sont des images MEB de faciès de rupture montrant respectivement des facettes de clivage, des grains équiaxes et des cupules ductiles. Les facettes de clivage (figure 12) et les grains équiaxes (figure 13 - qui apparaissent à partir de 500°C) qui ont été décrits dans ce qui précède sont facilement identifiables. Les cupules ductiles (figure 14) sont aussi clairement identifiables. Théoriquement, elles ne devraient pas apparaître sur des faciès de rupture de pièces de turbomachine en TiAl car ces cupules n'apparaissent qu'à partir d'une température de 800°C et les pièces en TiAl de turbomachine ne sont en général pas utilisées à des températures supérieures à 800°C. Toutefois, dans le cas où ces cupules apparaitraient sur un faciès de rupture ou de fissure d'une pièce de turbomachine, cela signifierait que cette pièce a été soumise à une surchauffe anormale (supérieure à 800°C). L'analyse de la présence de ces cupules ductiles à la surface d'un faciès de rupture ou de fissure participe donc à l'évaluation de la température vue par la pièce en fonctionnement.

## Revendications

1. Procédé d'analyse d'un faciès (10) de rupture ou de fissure d'une pièce métallique (12) de turbomachine, en particulier en matériau à rupture totalement fragile, tel un alliage à base de TiAl, ledit faciès correspondant au plan de rupture ou au plan de fissuration avant ouverture en laboratoire pour une pièce fissurée non rompue , **caractérisé en ce qu'**il comprend au moins l'une des étapes consistant à :
a) repérer sur le faciès la position et l'orientation de facettes de clivage (16), de façon à identifier une zone d'amorçage (26) de la rupture ou fissure et à déterminer le sens de propagation de cette rupture ou fissure,
b) examiner le faciès et détecter les zones de présence de grains équiaxes (42) et/ou de grains lamellaires (44), de façon à évaluer la température à laquelle la rupture ou fissure est intervenue, et
c) comparer la ou les colorations thermiques du faciès avec celles d'échantillons d'un nuancier (49) de coloration thermique, ces échantillons étant réalisés dans le même matériau que la pièce et étant soumis à des traitements thermiques oxydants à des températures et pendant des durées prédéterminées, de façon à évaluer la vitesse de propagation de la rupture ou fissure,
les étapes a), b) et c) étant effectuées dans un ordre quelconque.

2. Procédé selon la revendication 1, **caractérisé en ce que** l'étape a) consiste en outre à déterminer au moins un défaut (25) géométrique ou métallurgique pouvant expliquer l'apparition de la fissure ou rupture.

3. Procédé selon l'une des revendications précédentes, **caractérisé en ce qu'**il consiste, à l'étape a), à repérer et tracer directement sur une image vidéo du faciès la position et l'orientation des facettes de clivage (16).

4. Procédé selon l'une des revendications précédentes, **caractérisé en ce qu'**au moins une des étapes a) et b) est réalisée à la loupe binoculaire et/ou au moyen d'un système d'imagerie par microscopie électronique à balayage.

5. Procédé selon l'une des revendications précédentes, **caractérisé en ce que** l'étape b) consiste en outre à déterminer si la rupture ou fissure a eu lieu à chaud en service, ou à froid en fabrication.

6. Procédé selon l'une des revendications précédentes, **caractérisé en ce qu'**il consiste, à l'étape b), à déterminer si la rupture ou la fissure est intervenue à plus ou moins que 500°C.

7. Procédé selon l'une des revendications précédentes, **caractérisé en ce qu'**il consiste, à l'étape b), à évaluer la température par une estimation de la densité des grains équiaxes sur le faciès.

8. Procédé selon l'une des revendications précédentes, **caractérisé en ce qu'**il consiste en outre, à l'étape b), à examiner le faciès et détecter les zones de présence de cupules ductiles.

9. Procédé selon l'une des revendications précédentes, **caractérisé en ce que**, à l'étape c), avant la soumission des échantillons aux traitements thermiques, une entaille (50) est réalisée dans chaque échantillon qui est ensuite soumis à des contraintes pour générer une fissure ou rupture au niveau de cette entaille.

10. Procédé selon l'une des revendications précédentes, **caractérisé en ce que** l'étape c) consiste en outre à comparer les colorations thermiques d'une surface de la pièce avec celles des échantillons précités, de façon à évaluer le niveau de température atteint par la pièce.

## Patentansprüche

1. Verfahren zur Analyse eines Bruch- oder Risserscheinungsbildes (10) eines Metallstücks (12) einer Strömungsmaschine, insbesondere aus einem extrem fragilen Bruchmaterial wie z. B. einer Legierung auf Basis von TiAl, wobei das Erscheinungsbild zu einer Bruchebene oder einer Rissbildungsebene vor dem Öffnen im Labor für ein nicht gebrochenes, gerissenes Stück korrespondiert, **dadurch gekennzeichnet, dass** es mindestens einen der Schritte umfasst, die aus folgendem bestehen:
a) Auffinden, auf dem Erscheinungsbild, der Position und der Ausrichtung von Spaltflächen (16), indem ein Auslösungsbereich (26) des Bruchs oder des Risses identifiziert wird und indem die Ausbreitungsrichtung dieses Bruchs oder Risses bestimmt wird,
b) Untersuchen des Erscheinungsbilds und Detektieren der Bereiche, in welchen äquiaxiale Körner (42) und/oder lamellare Körner (44) vorhanden sind, indem die Temperatur ausgewertet wird, bei welcher der Bruch oder der Riss eingetreten ist, und
c) Vergleichen der Anlauffarbe oder der Anlauffarben des Erscheinungsbilds mit solchen von Proben einer Farbpalette (49) für Anlauffarben, wobei die Proben in dem gleichen Material wie das Stück verwirklicht sind und thermischen Oxidationsbehandlungen bei vorbestimmten Temperaturen und während vorbestimmter Zeitdauern unterzogen sind, indem die Ausbreitungsgeschwindigkeit des Bruchs oder Risses ausgewertet wird, wobei die Schritte a), b) und c) in beliebiger Reihenfolge durchgeführt werden.

2. Verfahren nach Anspruch 1, **dadurch gekennzeichnet, dass** der Schritt a) außerdem darin besteht, mindestens einen geometrischen oder metallurgischen Fehler (25) zu bestimmen, der das Auftreten des Bruches oder des Risses erklären kann.

3. Verfahren nach einem der vorhergehenden Ansprüche, **dadurch gekennzeichnet, dass** es in dem Schritt a) darin besteht, direkt auf einem Videobild des Erscheinungsbilds die Position und die Ausrichtung der Spaltflächen (16) aufzufinden und zu verfolgen.

4. Verfahren nach einem der vorhergehenden Ansprüche, **dadurch gekennzeichnet, dass** zumindest einer der Schritte a) und b) mit einer binokularen Lupe und/oder mit einem Mittel eines bildgebenden Systems durch Elektronenmikroskopie mit einer Abtastung erfolgt.

5. Verfahren nach einem der vorhergehenden Ansprüche, **dadurch gekennzeichnet, dass** der Schritt b) außerdem darin besteht, zu bestimmen, ob der Bruch oder der Riss bei Wärme während des Betriebs oder bei Kälte während der Herstellung erfolgt ist.

6. Verfahren nach einem der vorhergehenden Ansprüche, **dadurch gekennzeichnet, dass** es im Schritt b) darin besteht, zu bestimmen, ob der Bruch oder der Riss bei mehr oder bei weniger als 500 °C eingetreten ist.

7. Verfahren nach einem der vorhergehenden Ansprüche, **dadurch gekennzeichnet, dass** es in dem Schritt b) darin besteht, die Temperatur durch eine Schätzung der Dichte der äquiaxialen Körner auf dem Erscheinungsbild auszuwerten.

8. Verfahren nach einem der vorhergehenden Ansprüche, **dadurch gekennzeichnet, dass** es außerdem im Schritt b) darin besteht, das Erscheinungsbild zu untersuchen und die Bereiche zu detektieren, in welchen duktile Vertiefungen vorhanden sind.

9. Verfahren nach einem der vorhergehenden Ansprüche, **dadurch gekennzeichnet, dass** im Schritt c), bevor die Proben Wärmebehandlungen unterzogen werden, eine Kerbe (50) in jeder Probe erzeugt wird, welche anschließend Belastungen unterzogen wird, um einen Riss oder einen Bruch in Höhe dieser Kerbe zu erzeugen.

10. Verfahren nach einem der vorhergehenden Ansprüche, **dadurch gekennzeichnet, dass** der Schritt c) außerdem darin besteht, die Anlauffarben einer Oberfläche des Stücks mit denjenigen der genannten Proben zu vergleichen, indem die von dem Stück erreichte Temperaturhöhe ausgewertet wird.

## Claims

1. Method for analysing a fracture or crack face (10) of a metal part (12) of a turbine engine, in particular made of extremely fragile fracture material, such as a TiAl-based alloy, said face corresponding to the fracture surface or to the cracking surface before opening in the laboratory for an unbroken cracked part, **characterised in that** it comprises at least one of the steps consisting in:
a) identifying on the face the position and orientation of cleavage facets (16) so as to identify an initiation region (26) of the fracture or crack and to determine the direction of propagation of said fracture or crack,
b) examining the face and detecting the regions where equiaxed grains (42) and/or lamellar grains (44) are present, so as to evaluate the temperature at which the fracture or crack was made, and
c) comparing the heat tint(s) of the face with those of samples from a heat tint colour chart (49), said samples being produced from the same material as the part and being subjected to oxidising heat treatments at predetermined temperatures and for predetermined periods of time, so as to evaluate the speed of propagation of the fracture or crack,
steps a), b) and c) being carried out in any order.

2. Method according to claim 1, **characterised in that** step a) further consists in determining at least one geometrical or metallurgical fault (25) which is able to explain the appearance of the crack or fracture.

3. Method according to any of the preceding claims, **characterised in that** it consists, in step a), in identifying and directly tracing, in a video image of the face, the position and orientation of the cleavage facets (16).

4. Method according to any of the preceding claims, **characterised in that** at least one of steps a) and b) is carried out using a binocular loupe and/or by means of a scanning electron microscopy imaging system.

5. Method according to any of the preceding claims, **characterised in that** step b) further consists in determining whether the fracture or crack took place when hot during use or when cold during manufacture.

6. Method according to any of the preceding claims, **characterised in that** it consists, in step b), in determining whether the fracture or crack was made above or below 500 °C.

7. Method according to any of the preceding claims, **characterised in that** it consists, in step b), in evaluating the temperature by estimating the density of the equiaxed grains on the face.

8. Method according to any of the preceding claims, **characterised in that** it further consists, in step b), in examining the face and detecting the regions where ductile dimples are present.

9. Method according to any of the preceding claims, **characterised in that**, in step c), before the samples are subjected to heat treatments, a notch (50) is made in each sample which is then subjected to stresses to produce a fracture or crack in the region of said notch.

10. Method according to any of the preceding claims, **characterised in that** step c) further consists in comparing the heat tints on a surface of the part with those of the above-mentioned samples so as to evaluate the temperature level reached by the part.
